# EUROPEAN PATENT SPECIFICATION

(11) **EP 1 854 405 B1**
(45) Date of publication and mention of the grant of the patent: **26.06.2013**
(21) Application number: 07251929.1
(22) Date of filing: 10.05.2007
(51) Int. Cl.: A61B 5/06, G01B 7/004

(54) **Low-profile location pad**
Niedrigprofil-Positionsblock
Plaquette de localisation à faible profil

(30) Priority: 11.05.2006 US 382830
(43) Date of publication of application: 14.11.2007
(73) Proprietor: Biosense Webster, Inc., Diamond Bar, CA 91765 (US)
(72) Inventor: Govari, Assaf, Haifa 34400 (IL); Altmann, Andres Claudio, Haifa 34614 (IL); Ephrath, Yaron, Karkur 37501 (IL)
(74) Representative: Tunstall, Christopher Stephen

(56) References cited:
- EP-A- 1 481 635
- EP-A1- 1 174 082
- WO-A-96/05768
- WO-A-96/41119
- WO-A-97/00043
- US-A- 5 711 299
- US-B1- 6 773 393

## Description

### FIELD OF THE INVENTION

The present invention relates generally to position tracking systems, and particularly to location pads used in magnetic position tracking.

### BACKGROUND OF THE INVENTION

Various methods and systems are known in the art for tracking the coordinates of objects involved in medical procedures. Some of these systems use magnetic field measurements. For example, U.S. Patents 5,391,199 and 5,443,489 describe systems in which the coordinates of an intrabody probe are determined using one or more field transducers. Such systems are used for generating location information regarding a medical probe, such as a catheter. A position sensor, such as a coil, is placed in the probe and generates signals in response to externally-applied magnetic fields. The magnetic fields are generated by magnetic field transducers, such as radiator coils, fixed to an external reference frame in known, mutually-spaced locations. The CARTO system, for example, produced by Biosense Webster Inc. (Diamond Bar, California), operates in this manner.

Additional methods and systems that relate to magnetic position tracking are also described, for example, in PCT Patent Publication WO 96/05768, U.S. Patents 6,690,963, 6,484,118, 6,239,724, 6,618,612 and 6,332,089, and U.S. Patent Application Publications 2002/0065455 A1, 2003/0120150 A1 and 2004/0068178 A1.

These publications describe methods and systems that track the position of intrabody objects such as cardiac catheters, orthopedic implants and medical tools used in different medical procedures.

In some of the references cited above, the field transducers that generate the magnetic fields comprise field generating coils arranged in a coplanar, triangular arrangement. Another planar arrangement is described in U.S. Patent 6,615,155. The patent describes a system for tracking a moving object having a single field sensor using a set of nonoverlapping planar loop antennas.

WO 97/00043 discloses a system for navigating a catheter probe through a body cavity. The system includes a sensing coil affixed to a distal end of the probe. Magnetic fields are projected into the body cavity to induce voltage signals in the sensing coil that are sufficient to describe the orientation and position of the probe. A set of magnetic coils each generates a substantially uniform field in a single respective dimension.

WO 96/05768 discloses a locating system for determining the location and orientation of an invasive medical instrument relative to a reference frame. The system comprising a plurality of field generators which generate known, distinguishable fields in response to drive signals; a plurality of sensors situated in the invasive medical instrument proximate the distal end thereof which generate sensor signals in response to said fields; and a signal processor which has an input for a plurality of signals corresponding to said drive signals and said sensor signals and which produces the three location coordinates and three orientation coordinates of a point on the invasive medical instrument.

EP 1 174 082 discloses a system for determining the position of a medical device having a sensor. The system comprises a plurality of field radiators wherein each field radiator has a plurality of radiator elements. Each radiator element generates a magnetic field such that each magnetic field is distinct from one another due to the use of a different frequency. The magnetic fields are measured by the sensor. The field radiators and the sensor are connected to a signal processor. The signal processor includes an initial position estimator for establishing an initial position estimate for the sensor; a magnetic field calculator for calculating the magnetic field at the initial position estimate; a steepest descent calculator for calculating a steepest descent of the calculated magnetic field to the measured magnetic field; and a new position estimate calculator for calculating a new position estimate of said sensor based on said steepest descent. The signal processor determines the position of the sensor when the new position estimate of the sensor is within the desired range of accuracy for the system.

### SUMMARY OF THE INVENTION

The present invention provides a location pad, a magnetic position tracking system and a method as claimed hereinafter. Some embodiments of the present invention provide location pads comprising multiple magnetic field generators having a low profile (i.e., a reduced height dimension). This arrangement is advantageous in a number of medical position tracking applications. For example, a thin horizontal array of field generators can be conveniently placed on an operating or catheterization table, directly underneath a region of interest of a patient's body. A position sensor in a medical probe, such as a catheter, or other object in the body senses the fields produced by the field generators and outputs signals that are processed to determine coordinates of the probe or other object.

The position tracking system may assist a physician in performing cardiac catheterization procedures. A low profile location pad is placed on top of the catheterization table, underneath the patient's torso. A position sensor is fitted in the catheter inserted into the patient's heart. An additional reference position sensor may be attached to the patient's body, typically on the patient' back. Both sensors, and in particular the reference sensor, are adjacent to the plane of the location pad. Using the methods and systems described herein, the position tracking system can accurately track the positions of the catheter and the reference sensor.

A miniature magnetic field generator may be attached to the catheter (or other object in the body), and the coils or other antennas in the location pad are used to sense the magnetic field and thus to determine the coordinates of the catheter (or other object). The novel principles of the design of the location pad are equally applicable in arrangements in which the location pad serves as a receiver as in arrangements in which it generates the magnetic fields.

The one or more field generators may include two or more field generators positioned in a common plane and oriented so that the respective magnetic fields are perpendicular to the plane, and the system includes an auxiliary field generator oriented so as to generate an auxiliary magnetic field having a component parallel to the plane at locations within the common plane.

The system may include a reference position sensor, which is attached to the body of the patient at a reference location adjacent to the common plane and is arranged to sense the auxiliary magnetic field and the magnetic fields generated by the two or more field generators so as to measure a position of the reference location. The reference position sensor may be arranged to accurately measure a vertical displacement of the reference location based on the sensed auxiliary magnetic field.

The system may include a processor, which is arranged to calculate a position of the invasive medical device with respect to the body irrespective of patient movements based on magnetic field measurements provided by the position sensor fixed to the invasive medical device and by the reference position sensor.

The one or more field generators may include two or more field generators positioned at known angles with respect to one another.

The present invention will be more fully understood from the following detailed description of the embodiments thereof, taken together with the drawings in which:

### BRIEF DESCRIPTION OF THE DRAWINGS

Fig. 1 is a schematic, pictorial illustration of a magnetic position tracking system, in accordance with an embodiment of the present invention;
Figs. 2A and 2B are diagrams that schematically illustrate a low profile location pad, in accordance with an embodiment of the present invention; and
Fig. 3 is a flow chart that schematically illustrates a method for magnetic position tracking, in accordance with an embodiment of the present invention.

### DETAILED DESCRIPTION OF EMBODIMENTS

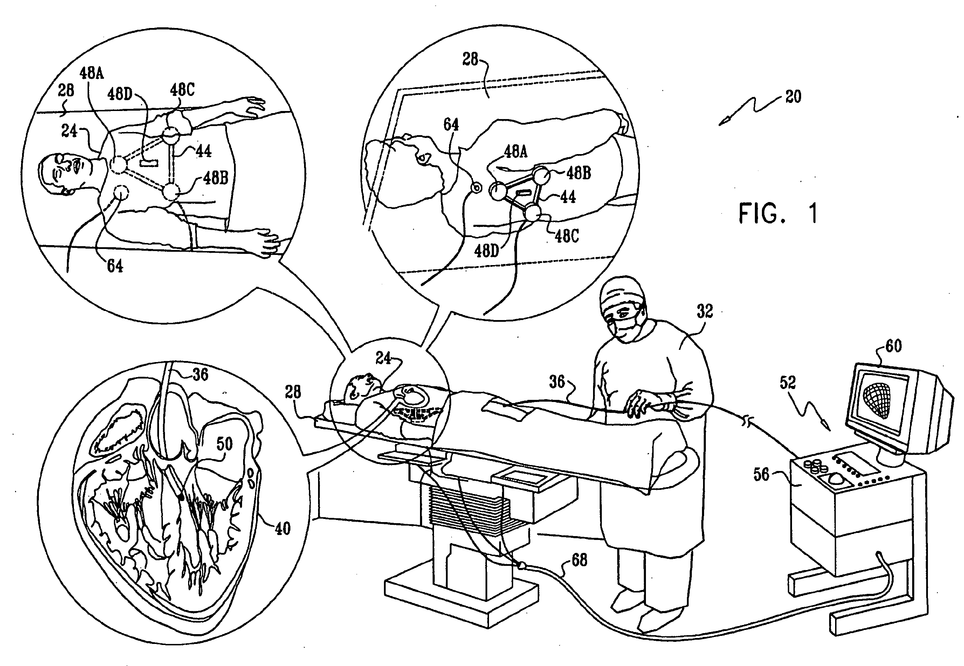

Fig. 1 is a schematic, pictorial illustration of a magnetic position tracking system 20 used in cardiac catheterization applications, in accordance with an embodiment of the present invention. A patient 24 lies on a catheterization table 28. A physician 32 inserts a catheter 36 into a chamber of a heart 40 of the patient. System 20 determines and displays the position and orientation coordinates of catheter 36 inside the heart.

System 20 comprises a location pad 44, which comprises one or more field generators, such as field generating coils. In the exemplary configuration of Fig. 1, pad 44 comprises four field generating coils 48A...48D. The location pad is placed on top of the catheterization table under the patient's torso, such that coils 48A...48D are located in fixed, known positions external to the patient. The field generating coils generate magnetic fields in a predefined working volume around heart 40.

The operation of system 20 is similar to that of the above-mentioned CARTO system, except that CARTO uses a bulky arrangement of field generator coils, which must be specially mounted beneath the catheterization table. Placing a thin location pad on the table obviates the need for special mounting and permits the pad to be moved freely from table to table. Placing the location pad on top of the table also increases the volume in which accurate position measurements can be performed, and may sometimes enable reducing the size of the position sensor. Additionally, when placed on top of the table, the location pad is typically further away from interfering objects, such as C-arm fluoroscopes, which may degrade the accuracy of position measurements.

A position sensor 50 fitted in the distal end of catheter 36 senses the magnetic fields in its vicinity. The position sensor produces and transmits, in response to the sensed fields, position signals to a console 52. The console comprises a tracking processor 56 that calculates the location and orientation of catheter 36 with respect to coils 48A...48D, based on the position signals sent by position sensor 50. Typically, the console also drives coils 48A...48D to generate the appropriate magnetic fields. The location and orientation coordinates of catheter 36 are displayed to the physician using a display 60.

In some embodiments, a reference position sensor 64 is attached to the patient's back and is used for calibration purposes. By tracking the position of reference sensor 64, system 20 can measure movements of the patient with respect to location pad 44 and can thus separate these movements from the movement of catheter 36 inside the patient's body. In other words, by measuring the relative position of sensor 50 with respect to reference sensor 64, system 20 can accurately determine the position of the catheter in the heart, regardless of patient movements, due to shifting of the patient's body or to respiration, for example. Sensor 64 and location pad 44 are connected to console 52 using suitable cables 68.

In order to enable location pad 44 to be conveniently placed between the patient and the catheterization table, the location pad is produced having a low profile. The thickness of the pad does not exceed 3 cm, and is typically smaller than 2 cm.

In some situations, however, the low profile of the location pad may degrade the measurement accuracy of the system. For example, in order to minimize the thickness of location pad 44, three of the field generating coils denoted 48A...48C comprise low profile coils, wound in the horizontal plane in order to reduce their height dimension. As such, coils 48A...48C generate magnetic fields perpendicular to the horizontal plane. This arrangement of fields may produce inaccurate position measurements of position sensors located adjacent to the horizontal plane containing the field generator coils. In particular, it may be difficult to accurately measure vertical displacements of such sensors. As can be seen in the figure, reference position sensor 64 is located only slightly above the horizontal plane containing field generating coils 48A...48C (referred to as the plane of the location pad). Thus, sensor 64 may have poor sensitivity to vertical displacements when the measurements are based only on the fields generated by field generating coils 48A...48C.

In order to improve the measurement accuracy of system 20, and in particular vertical displacement measurements of reference position sensor 64, field generating coil 48D, referred to as an auxiliary coil, is added to location pad 44. Coils 48A...48C are referred to as primary field generators and the fields they generate are referred to as primary magnetic fields. Auxiliary coil 48D is wound in a plane perpendicular to the plane of the location pad, i.e., a plane that contains the vertical axis. As such, the auxiliary field generated by coil 48D is parallel to the plane of the location pad. Position signals produced by reference position sensor 64 due to the field generated by coil 48D will thus give an accurate indication of the vertical displacement of sensor 64, even though sensor 64 is adjacent to the plane of location pad 44. As will be shown below, coil 48D is wound so as to conform to the low profile of the location pad.

In alternative embodiments, the windings of coil 48D can be oriented at other angles with respect to the plane of the location pad, as long as the coil is not parallel with the plane. In other words, the field generated by coil 48D at locations within the plane of the location pad should have at least a component that is parallel to the plane. This component should be substantial enough to permit the vertical displacement of sensor 64 to be determined accurately to within the specified resolution limits of system 20. Further alternatively, the field generating coils can be tilted or otherwise oriented at known angles with respect to the horizontal plane, as long as they do not exceed the low profile dimensions of the pad.

In the example of Fig. 1, location pad 44 comprises a total of four field generating coils, out of which one coil comprises an auxiliary coil. In alternative embodiments, pad 44 may comprise any number of primary and auxiliary field generating coils arranged in any suitable configuration. Further alternatively, pad 44 may comprise a single field generating coil and catheter 36 comprises multiple position sensors positioned at known relative offsets and/or orientations, so as to enable tracking of the catheter location.

In some embodiments, auxiliary coil 48D may be located externally to location pad 44, such as next to the patient's feet. In such embodiments, there is no strict requirement as to the mechanical dimensions of the auxiliary coil. The auxiliary coil should still be positioned and oriented so that the field it generates has a component parallel to the plane of the location pad.

Although Fig. 1 shows a system for cardiac catheterization, low profile location pad 44 can be used in any other position tracking application, such as for tracking orthopedic implants and medical tools, as described in the background references cited above. In the example of Fig. 1 the location pad is placed horizontally and has a reduced height or vertical dimension. The methods and devices described herein can be used to reduce any desired dimension of the location pad, as appropriate for the particular application.

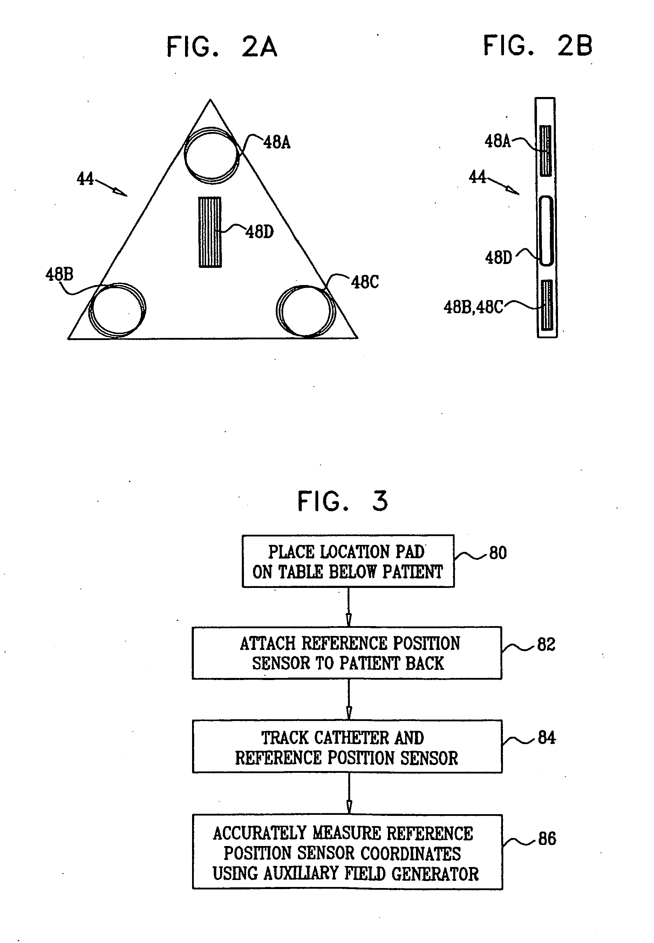

Figs. 2A and 2B are diagrams that schematically illustrate low profile location pad 44, in accordance with an embodiment of the present invention.

Fig. 2A is a top view of pad 44, showing primary coils 48A...48C arranged in a co-planar triangular configuration. The coils typically comprise air-wound coils, wound without a solid core or bobbin, so as to minimize their thickness. Alternatively, coils having cores that do not significantly increase the thickness of the pad can also be used. The distance between the coils is typically in the range of several centimeters to several tens of centimeters, although other distances can also be used. In the present example, the axis of auxiliary coil 48D is parallel to the plane of the location pad. Coil 48D is shown in the middle of the location pad, although it may alternatively be mounted at any other convenient location.

Fig. 2B is a side view of pad 44, showing coils 48A...48D conforming to the overall low profile of the pad. In particular, coil 48D is shown to be wound using long and narrow windings, so as not to protrude beyond the thickness of primary coils 48A...48C. The four coils may be fixed in their respective positions using a baseplate, frame or any other suitable fixture.

Fig. 3 is a flow chart that schematically illustrates a method for magnetic position tracking during a catheterization procedure, in accordance with an embodiment of the present invention. The method begins by placing location pad 44 on the catheterization table underneath the patient, at a pad positioning step 80. The physician attaches reference position sensor 64 to the patient's back, at a reference positioning step 82.

During the catheterization procedure, system 20 tracks position sensor 50 in catheter 36, as well as reference position sensor 64, at a tracking step 84. The four field generating coils 48A...48D generate respective magnetic fields, which are sensed by position sensors 50 and 64. Tracking processor 56 accepts the position signals produced by sensors 50 and 64 responsively to the fields and calculates the position (location and orientation) coordinates of the two sensors. In particular, processor 56 accurately tracks the position of reference position sensor 64, at a reference tracking step 86. Using the field generated by auxiliary coil 48D, processor 56 is able to accurately determine the position of sensor 64, even though it is adjacent to the plane of the location pad.

Although the embodiments described hereinabove relate to a system in which a magnetic field is generated outside the patient's body and sensed by a position sensor in the body, in alternative embodiments the position sensor may be replaced by a miniature field generator in catheter 36, and coils 48A...48D may be used instead to sense the field generated by the field generator in the catheter (and possibly by a reference field generator in place of sensor 64). Furthermore, although these embodiments refer mainly to a low profile location pad to be used in a magnetic position tracking system, the principles of the present invention can also be used in additional applications, such as in wireless power transfer applications in which electrical power is transferred to a low profile antenna pad.

It will thus be appreciated that the embodiments described above are cited by way of example, and that the present invention is not limited to what has been particularly shown and described hereinabove. Rather, the scope of the present invention includes both combinations and sub-combinations of the various features described hereinabove, as well as variations and modifications thereof which would occur to persons skilled in the art upon reading the foregoing description and which are not disclosed in the prior art.

## Claims

1. A location pad (44) for use in a magnetic position tracking system (20), comprising:
one or more primary field generators (48A, 48B, 48C), which are arranged in a common plane, wherein the one or more field generators (48A, 48B, 48C) comprise primary field generating coils wound in parallel to the common plane so as to generate respective magnetic fields perpendicular to the common plane; and
an auxiliary field generator (48D), wherein the auxiliary field generator (48D) comprises an auxiliary field generating coil wound in a plane perpendicular to the common plane so as to generate an auxiliary magnetic field that is parallel to the common plane, wherein the auxiliary field generator is wound such that the windings are longer in the common plane than they are in a direction perpendicular to the common plane, so as not to protrude beyond the thickness of the primary field generating coils in the direction perpendicular to the common plane;
wherein the one or more primary field generators (48A, 48B, 48C) and the auxiliary field generator (48D) are arranged so that a thickness dimension of the location pad (44) is no greater than 3 centimeters.

2. A magnetic position tracking system (20) for use in performing a medical procedure on a patient (24) who is positioned on an upper surface of a table (28), the system (20) comprising:
the location pad (44) of claim 1, which is positioned on the upper surface of the table (28) beneath the patient (24); and
a position sensor (50), which is fixed to an invasive medical device (36) for insertion into a body of the patient (28), and which is arranged to sense the auxiliary magnetic field and the magnetic fields generated by the one or more primary field generators (48A, 48B, 48C) so as to measure a position of the medical device (36) in the body.

3. The system according to claim 2, wherein the one or more primary field generators comprise two or more primary field generators.

4. The system according to claim 3, and comprising a reference position sensor (64), which is attached to the body of the patient (28) at a reference location adjacent to the common plane and is arranged to sense the auxiliary magnetic field and the magnetic fields generated by the two or more primary field generators (48A, 48B, 48C) so as to measure a position of the reference location.

5. The system according to claim 4, wherein the reference position sensor (64) is arranged to accurately measure a vertical displacement of the reference location based on the sensed auxiliary magnetic field.

6. The system according to claim 4, and comprising a processor (56), which is arranged to calculate a position of the invasive medical device (36) with respect to the body irrespective of patient (28) movements based on magnetic field measurements provided by the position sensor fixed to the invasive medical device and by the reference position sensor (64).

7. A method of positioning a location pad as part of a position tracking method, comprising:
positioning and orienting one or more primary field generators (48A, 488, 48C) in a common plane so as to generate respective primary magnetic fields perpendicular to the common plane, wherein the primary field generators (48A, 48B, 48C) comprise primary field generating coils wound in parallel to the common plane; and
positioning and orienting an auxiliary field generator (48D) to generate an auxiliary magnetic field that is parallel to the common plane, wherein the auxiliary field generator (48D) comprises an auxiliary field generating coil wound in a plane perpendicular to the common plane, wherein the auxiliary field generator is wound such that the windings are longer in the common plane than they are in a direction perpendicular to the common plane, so as not to protrude beyond the thickness of the primary field generating coils in the direction perpendicular to the common plane; and
wherein the primary (48A, 48B, 48C) and auxiliary field (48D) generators are arranged so that a thickness dimension of the location pad is no greater than 3 centimeters.

## Patentansprüche

1. Positionsblock (44) zur Verwendung in einem magnetischen Positionsverfolgungssystem (20), umfassend:
ein oder mehr primäre Feldgeneratoren (48A, 48B, 48C), die in einer gemeinsamen Ebene angeordnet sind, worin der eine oder die mehreren Feldgeneratoren (48A, 48B, 48C) primäre Felderzeugungsspulen umfassen, die parallel zur gemeinsamen Ebene gewickelt sind, um jeweils Magnetfelder lotrecht zur gemeinsamen Ebene zu erzeugen; und
einen Hilfsfeldgenerator (48D) worin der Hilfsfeldgenerator (48D) eine Hilfsfelderzeugungsspule umfasst, die in einer Ebene lotrecht zur gemeinsamen Ebene gewickelt ist, um ein Hilfsmagnetfeld zu erzeugen, das zur gemeinsamen Ebene parallel ist, worin der Hilfsfeldgenerator so gewickelt ist, dass die Windungen in der gemeinsamen Ebene länger sind als in einer Richtung lotrecht zur gemeinsamen Ebene, damit sie nicht über die Dicke der primären Felderzeugungsspulen in der Richtung lotrecht zur gemeinsamen Ebene vorstehen;
worin der eine oder die mehreren primären Feldgeneratoren (48A, 48B, 48C) und der Hilfsfeldgenerator (48D) so angeordnet sind, dass eine Dickenabmessung des Positionsblocks (44) nicht größer als 3 Zentimeter ist.

2. Magnetisches Positionsverfolgungssystem (20) zur Verwendung bei der Durchführung eines medizinischen Verfahrens an einem Patienten (24), der auf einer Oberfläche eines Tisches (28) positioniert ist, wobei das System (20) Folgendes umfasst:
den Positionsblock (44) nach Anspruch 1, der auf der Oberfläche des Tisches (28) unter dem Patienten (24) angeordnet ist; und
einen Positionssensor (50), der zur Einführung in einen Körper des Patienten (28) an einer invasiven medizinischen Vorrichtung (36) befestigt ist und so angeordnet ist, dass er das Hilfsmagnetfeld und die von dem einen oder den mehreren primären Feldgeneratoren (48A, 48B, 48C) erzeugten Magnetfelder zur Messung einer Position der medizinischen Vorrichtung (36) im Körper misst.

3. System nach Anspruch 2, worin der eine oder die mehreren primären Feldgeneratoren zwei oder mehr primäre Feldgeneratoren umfassen.

4. System nach Anspruch 3, umfassend einen Referenzpositionssensor (64), der am Körper des Patienten (28) an einer Bezugsstelle neben der gemeinsamen Ebene befestigt und so angeordnet ist, dass er das Hilfsmagnetfeld und die von dem einen oder den mehreren primären Feldgeneratoren (48A, 48B, 48C) erzeugten Magnetfelder zur Messung einer Position der Bezugsstelle misst.

5. System nach Anspruch 4, worin der Referenzpositionssensor (64) zur genauen Messung einer vertikalen Verschiebung der Bezugsstelle auf Basis des gemessenen Hilfsmagnetfelds angeordnet ist.

6. System nach Anspruch 4, umfassend einen Prozessor (56), der zur Berechnung einer Position der invasiven medizinischen Vorrichtung (36) relativ zum Körper unabhängig von Bewegungen des Patienten (28) auf Basis von Magnetfeldmessungen, die von dem an der invasiven medizinischen Vorrichtung befestigten Positionssensor und vom Referenzpositionssensor (64) bereitgestellt werden, angeordnet ist.

7. Verfahren zur Positionierung eines Positionsblocks als Teil eines Positionsverfolgungsverfahrens, umfassend:
Positionierung und Orientierung von einem oder mehreren primären Feldgeneratoren (48A, 48B, 48C) in einer gemeinsamen Ebene zur Erzeugung von jeweiligen primären Magnetfeldern lotrecht zu der gemeinsamen Ebene, worin die primären Feldgeneratoren (48A, 48B, 48C) primäre Felderzeugungsspulen umfassen, die parallel zur gemeinsamen Ebene gewickelt sind; und
Positionierung und Orientierung eines Hilfsfeldgenerators (48D) zur Erzeugung eines Hilfsmagnetfelds, das parallel zur gemeinsamen Ebene ist, worin der Hilfsfeldgenerator (48D) eine Hilfsfelderzeugungsspule umfasst, die in einer Ebene lotrecht zur gemeinsamen Ebene gewickelt ist, worin der Hilfsfeldgenerator so gewickelt ist, dass die Windungen in der gemeinsamen Ebene länger sind als in einer Richtung lotrecht zur gemeinsamen Ebene, damit sie nicht über die Dicke der primären Felderzeugungsspulen in der Richtung lotrecht zur gemeinsamen Ebene vorstehen; und
worin der eine oder die mehreren primären Feldgeneratoren (48A, 48B, 48C) und der Hilfsfeldgenerator (48D) so angeordnet sind, dass eine Dickenabmessung des Positionsblocks nicht größer als 3 Zentimeter ist.

## Revendications

1. Plaquette (44) de localisation destinée à être utilisée dans un système (20) magnétique de suivi d'une position, comportant :
un ou plusieurs générateurs (48A, 48B, 48C) de champs primaires, qui sont disposés dans un plan commun, dans lequel ledit un ou plusieurs générateurs (48A, 48B, 48C) de champs comporte des bobines de génération de champs primaires enroulées parallèlement au plan commun de façon à générer des champs magnétiques respectifs perpendiculaires au plan commun ; et
un générateur (48D) de champ auxiliaire, dans lequel le générateur (48D) de champ auxiliaire comporte une bobine de génération d'un champ auxiliaire enroulée dans un plan perpendiculaire au plan commun de façon à générer un champ magnétique auxiliaire qui est parallèle au plan commun, dans lequel le générateur de champ auxiliaire est enroulé de telle sorte que les enroulements sont plus longs dans le plan commun qu'ils ne le sont dans une direction perpendiculaire au plan commun, de manière à ne pas faire saillie au-delà de l'épaisseur des bobines de génération de champs primaires dans la direction perpendiculaire au plan commun ;
dans lequel ledit un ou plusieurs générateurs (48A, 48B, 48C) de champs primaires et le générateur (48D) de champ auxiliaire sont disposés de façon à ce qu'une dimension d'épaisseur de la plaquette (44) de localisation ne soit pas supérieure à 3 centimètres.

2. Système (20) magnétique de suivi d'une position destiné à être utilisé pour réaliser une intervention médicale sur un patient (24) qui est positionné sur une surface supérieure d'une table (28), le système (20) comportant :
la plaquette (44) de localisation selon la revendication 1, qui est positionnée sur la surface supérieure de la table (28) sous le patient (24) ; et
un capteur (50) de position, qui est fixé à un dispositif (36) médical invasif destiné à être introduit dans le corps du patient (28), et qui est disposé de manière à capter le champ magnétique auxiliaire et les champs magnétiques générés par ledit un ou plusieurs générateurs (48A, 48B, 48C) de champs primaires de façon à mesurer une position du dispositif (36) médical dans le corps.

3. Système selon la revendication 2, dans lequel ledit un ou plusieurs générateurs de champs primaires comporte deux générateurs de champs primaires ou plus.

4. Système selon la revendication 3, et comportant un capteur (64) de position de référence, qui est fixé au corps du patient (28) à un emplacement de référence adjacent au plan commun et qui est disposé de manière à capter le champ magnétique auxiliaire et les champs magnétiques générés par les deux générateurs (48A, 48B, 48C) de champs primaires ou plus de façon à mesurer une position de l'emplacement de référence.

5. Système selon la revendication 4, dans lequel le capteur (64) de position de référence est disposé de manière à mesurer avec précision un déplacement vertical de l'emplacement de référence sur la base du champ magnétique auxiliaire capté.

6. Système selon la revendication 4, et comportant un processeur (56), qui est disposé de manière à calculer une position du dispositif (36) médical invasif par rapport au corps indépendamment des mouvements du patient (28) sur la base des mesures des champs magnétiques fournies par le capteur de position fixé au dispositif médical invasif et par le capteur (64) de position de référence.

7. Procédé de positionnement d'une plaquette de localisation faisant partie d'un procédé de suivi d'une position, consistant à :
positionner et orienter un ou plusieurs générateurs (48A, 48B, 48C) de champs primaires dans un plan commun de manière à générer des champs magnétiques primaires respectifs perpendiculaires au plan commun, dans lequel les générateurs (48A, 48B, 48C) de champs primaires comprennent des bobines de génération de champs primaires enroulées parallèlement au plan commun ; et
positionner et orienter un générateur (48D) de champ auxiliaire pour générer un champ magnétique auxiliaire qui est parallèle au plan commun, dans lequel le générateur (48D) de champ auxiliaire comporte une bobine de génération d'un champ auxiliaire enroulée dans un plan perpendiculaire au plan commun, dans lequel le générateur de champ auxiliaire est enroulé de telle sorte que les enroulements sont plus longs dans le plan commun qu'ils ne le sont dans une direction perpendiculaire au plan commun, de façon à ne pas faire saillie au-delà de l'épaisseur des bobines génération des champs primaires dans la direction perpendiculaire au plan commun ; et
dans lequel les générateurs des champs primaires (48A, 48B, 48C) et auxiliaire (48D) sont disposés de façon à ce qu'une dimension d'épaisseur de la plaquette de localisation ne soit pas supérieure à 3 centimètres.
